Europäisches Patentamt
European Patent Office
Office européen des brevets

(19)

(11) Publication number: **0 408 108 A2**

(12) **EUROPEAN PATENT APPLICATION**

(21) Application number: 90201744.1

(22) Date of filing: 29.06.90

(51) Int. Cl.5: A61K 31/19, A61K 31/215, A61K 31/275

(30) Priority: 14.07.89 HU 357289

(43) Date of publication of application: 16.01.91 Bulletin 91/03

(84) Designated Contracting States: AT BE CH DE DK ES FR GB GR IT LI NL SE

(71) Applicant: RICHTER GEDEON VEGYESZETI GYAR R.T.
Gyömröi ut 19-21
H-1103 Budapest X(HU)

(72) Inventor: Fodor, Tamas
Népkötarsasag u.3
1061 Budapest(HU)
Inventor: Fischer, Janos
Bartok B. ut 32
1111 Budapest(HU)
Inventor: Dobay, Laszlo
Kertész u. 42-44
1073 Budapest(HU)
Inventor: Ezer, Elemér
Lupény u. 6-8
1026 Budapest(HU)
Inventor: Matuz, Judit
Ozgida u. 32
1025 Budapest(HU)
Inventor: Saghy, Katalin
Ulloi ut 60-62
1082 Budapest(HU)
Inventor: Szporny, Laszlo
Szabolcska M. u 7
1114 Budapest(HU)
Inventor: Hajos, György
Gabor u. 59
1026 Budapest(HU)
Inventor: Trischler, Ferenc
Uttöro u. 16
1171 Budapest(HU)

(74) Representative: Kupecz, Arpad et al
Octrooibureau Los en Stigter B.V. Postbox 20052
NL-1000 HB Amsterdam(NL)

(54) **Pharmaceutical compositions containing acrylic acid derivatives and their use in the medicine.**

(57) Known acrylic acid derivatives of formula

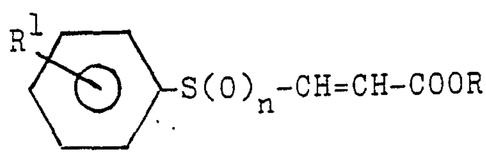

(I)

wherein
n is an integer of from 0 to 2,
R represents a hydrogen, an alkaline or an alkaline earth metal atom, or a $C_{1-4}$alkyl-group, and
$R^1$ represents a hydrogen or a halogen atom or a $C_{1-4}$-alkyl, a $C_{1-4}$alkoxy or a nitro group have useful pharmaceutical activities, in particular cytoprotective and optionally antiulcer activities. They can be used in the treatment and prevention of ulceration in the digestive tract of mammals, inclusive men.

EP 0 408 108 A2

# PHARMACEUTICAL COMPOSITIONS CONTAINING ACRYLIC ACID DERIVATIVES AND THEIR USE IN THE MEDICINE

The present invention is concerned with pharmacutical compositions comprising one or more pharmaceutically acceptable inert carriers and as active ingredient having cytoprotective and optionally gastric acid secretion inhibitory activity at least one compound of the formula

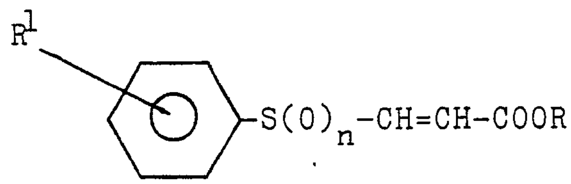

(I)

wherein
n is an integer of from 0 to 2,
R represents a hydrogen, an alkaline or an alkaline earth metal atom, or a $C_{1-4}$alkyl group, and $R^1$ represents a hydrogen or a halogen atom or a $C_{1-4}$-alkyl, a $C_{1-4}$alkoxy or a nitro group.

The compounds of formula (I), as well as their preparation and non-therapeutical use are described in a number of papers and patent specifications.

Those compounds of formula (i) wherein n is zero, and analogous compounds being different only in the meaning of R and $R^1$ are referred to hereinafter as phenylthiopropenoic acid derivatives.

Those compounds of formula (I) wherein n is 1, and analogous compounds being different only in the meaning of R and $R^1$ are referred to hereinafter as phenylsulfinylpropenoic acid derivatives.

Those compounds of formula (I) wherein n is 2, and analogous compounds being different only in the meaning of R and $R^1$ are referred to hereinafter as phenylsulfonylpropenoic acid derivatives.

US patent specification 2,532,291 describes the preparation of phenylthiopropenoic acid derivatives. No teaching is disclosed, however, concerning their use.

Japanese laid-open patent specifications 52-7919 and 52-7920 disclose the preparation of phenylthio-, phenylsulfinyl- and phenylsulfonylpropenoic acid derivatives and their alkaline metal salts. The compounds prepared in JP 52-7919 are declared as surface active agents.

Japanese laid-open patent specification 151,121 discloses the preparation of phenylthiopropenoic acid derivatives and their alkaline metal salts. These compounds are stated to be surface active agents and antibacterial agents and are suggested to be used as detergents, bactericidal or disintoxicating agents, furthermore as cream bases.

Japanese laid-open patent specification 151,123 describes the preparation of phenylsulfinyl- and phenylsulfonylpropenoic acid derivatives. Alkaline metal and alkaline earth metal salts of these compounds are also claimed but are not prepared. The use of these compounds as antibacterial agents, disintoxicating agents, antifungal agents against mould as well as antioxidants is suggested.

European patent specification 40359 describes certain phenylsulfinylpropenoic acid derivatives useful as intermediates in the preparation of dyestuffs.

Surprisingly we have found, that the compounds of formula (I) can also be used as human medicaments in particular for the pre- and after-treatment of patients having or prone to have ulceration.

A steadily increasing part of the population is involved in the ulceration of the digestive tract. Ulcer induces a very strong pain in its active stage and bleeding can also occur. According to the traditional medication, the primary object is to reduce the pain, then to promote the healing of the injured tissues. Traditional medicaments (e.g. Pyrenzepine, Cimetidine, Omeprazole, etc.) have been intended to achieve the above effects by the reduction of the gastric acid level and inhibition of the secretion of gastric acid, respectively. In case of proper medication and diet, ulcer is generally healed after 4 to 6 weeks. However, it happens frequently that ulcer relapses and the medication should be recommenced.

Recent investigations are focussed increasingly to the so-called cytoprotective compounds. These compounds increase the protective ability of the stomach thereby upon the administration of such compounds the probability of a relapse of ulcer is significantly reduced or the emergence of ulcer can be prevented in a subject susceptible to ulcer.

Compounds exerting inhibitory activity on gastric acid secretion in addition to their cytoprotective

activity are, of course, of particular interest.

Among the compounds of the present invention, there are several compounds having both cytoprotective and gastric acid secretion inhibitory activities. We have found, additionally, that the compounds of the invention exert prolonged action for both activities.

In the Tables below illustrating thre results of biological investigations the following abbreviations are used:

A: 3-phenylthio-2(Z)-propenoic acid
B: 3-phenylthio-2(E)-propenoic acid
C: 3-phenylsulfinyl-2(Z)-propenoic acid
D: 3-phenylsulfinyl-2(E)-propenoic acid
E: 3-phenylsulfonyl-2(Z)-propenoic acid
F: 3-phenylsulfonyl-2(E)-propenoic acid
G: magnesium bis[3-phenylthio-2(E)-propenoate]
H: magnesium bis[3-phenylsulfonyl-2(E)-propenoate]hydrate
I: methyl 3-phenylsulfonyl-2(E)-propenoate

The compounds of the invention were tested for their biological activity by the following methods.

1) Assay of gastric injuries induced by acidic alcohol

[A. Robert, Gastroenterology, 77 , 761-767 (1979)]

Female rats weighing about 120 to 150 g fasted for 24 hours were used in this test. Test compounds suspended with Tween 80 were given orally to the animals through an intragastric tube. After a certain period (pretreatment time) acidic alcohol was given through the intragastric tube at a dose or 0.5 ml per 100 g of body weight. The animals were sacrificed after 1 hour, their stomach was removed and incised along the great curve. The length of the reddish-brown strips (haemorrhagic lesions) was measured and the mean total length per stomach was calculated. The biological activity of the test compounds was given compared to the control group. The results are shown in Tables 1 and 2 below.

Table 1

| Compound | $ED_{50}$ p.o. (pretreatment: 30 min) |
|---|---|
| B | 31.0 mg/kg |
| D | 4.2 mg/kg |
| F | 3.4 mg/kg |
| H | 3.2 mg/kg |
| Reference compound: Sucralfat | 150 mg/kg |

Table 2

| Compound | % Inhibition at a dose of 10 mg/kg p.o. (pretreatment: 30 min) |
|---|---|
| C | 56 |
| D | 64 |
| E | 13 |
| F | 86 |
| H | 96 |
| I | 98 |

2)Assay of a chronic ulcer model induced by acetic acid

[Tagaki et al, Journal of Pharmacology 19 , 418-426 (1969)]

The assay was carried out in the following manner. Abdominal wall of femal rats previously fasted for 24 hours was incised under ether anaesthesia. A 20% solution of acetic acid (25 µl) was injected to the subserosus layer of the glandular part of the stomach near the pylorus. Thereafter the abdominal wall was closed and the animal was kept on normal diet, by providing feed and water ad libitum. The treatment was started 5 days after the operation and continued until the 10th day by administering the test compound once a day The animals were sacrificed 15 days after the operation and their stomach was removed. The extent of the ulcer was determined by measuring the diameter of the necrotic regions and calculating their total area. The curative effect of the tested compounds was calculated by the following formula in %:

$$\frac{\text{area of the ulcer}_{\text{controll}} - \text{area of the ulcer}_{\text{treated}}}{\text{area of the ulcer}_{\text{controll}}} \text{x100}$$

The results are shown in Table 3.

Table 3

| Compound | Dose | % Curative effect |
|---|---|---|
| F | 50 mg/kg p.o. | 37 |
| H | 30 mg/kg p.o. | 27 |
| I | 30 mg/kg p.o. | 73 |
| Reference compound: Sucralfat | 500 mg/kg p.o. | 55 |

3) Assay of the inhibition of gastric acid secretion by using pylorus ligature

[Shay et al., Gastroenterology, 5 , 43-61 (1945)]

Before ligating the pylorus the test compounds suspended with Tween were given orally at a volume of 0.5 ml per 100 mg of body weight to female Wistar rats fasted previously for 20 hours. The animals were sacrificed 4 hours after the operation, and the amount of the acid in the stomach was measured by titration with 0.01N sodium hydroxide solution in the presence of phenolphtalein indicator. The pH value of the content of stomach was measured by using a pH-meter (Radelkis, Type OP-211/1). The results are shown in Tables 4 and 5.

Table 4

| Compound | $ED_{50}$ p.o. (pretreatment: 30 min) |
|---|---|
| F | 12.0 mg/kg |
| H | 10.0 mg/kg |

Table 5

| Compound | % Inhibition in the amount of the acid at a dose of 10 mg/kg p.o. (pretreatment: 30 min) |
|---|---|
| C | 15 |
| D | 22 |
| F | 48 |
| G | 16 |
| H | 63 |
| I | 21 |

Therapeutic significance of the compounds according to the invention is further increased by the fact that they have bactericidal activity against Campylobacter pylori, the presence of which is a risk factor in the emergence of ulcers, or the healing of an ulcer of the digestive tract is influenced negatively by the presence of this bacterium [Internist, 29 , 745-754, (1988)].

The activity of the compounds of formula (I) against Campylobacter pylori was investigated by agar diffusion and agar dilution methods. The experiments were carried out with Campylobacter pylori cultures isolated from 5 different ulcerous patients. The MIC (minimal inhibitory concentration) values of compounds A and F were found to be 500 to 1000 µg/ml, while that of I was 250 to 270 µg/ml.

Toxicological data of the compounds of the invention are also beneficial. No death was observed when the compounds of the invention were given orally at a single dose of 1000 mg/kg of body weight.

According to a first aspect of the invention, there is provided a pharmaceutical composition containing as active ingredient at least one compound of formula

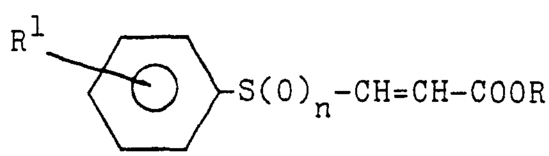

(I)

wherein
n is an integer of from 0 to 2,
R represents a hydrogen, an alkaline or an alkaline earth metal atom, or a $C_{1-4}$alkyl group, and
R1 represents a hydrogen or a halogen atom or a $C_{1-4}$-alkyl, a $C_{1-4}$alkoxy or a nitro group
and optionally one or more known active ingredient having no synergistic effect in the given combination, together with at least one pharmaceutically acceptable inert carrier and/or other excipient. These pharmaceutical compositions exert cytoprotective and optionally gastric acid secretion inhibitory activities, therefore they may be used for the prophylaxis and treatment of ulcerations in the digestive tract of mammals, inclusive human beings.

These pharmaceutical compositions may be prepared by admixing the active ingredient(s) with non-toxic, inert solid or liquid carriers and/or other excipients conventionally used in formulations for parenteral or enteral administration.

Suitable carriers are e.g. water, gelatin, lactose, starch, pectin, magnesium stearate, stearic acid, talc, vegetable oils such as peanut oil, olive oil, etc. The active ingredient may be formulated in a conventional manner e.g. to a solid composition such as tablet, lozenge, dragee, capsule, like gelatin capsule, pill, etc.

The pharmaceutical compositions of the invention optionally may contain one or more conventional excipients e.g. preservatives, stabilizing agents, wetting agents, emulgeators, etc.

These formulations may be prepared by any suitable method, e.g. in case of solid formulations by sieving, admixing, granulating and compressing the ingredients. The so-obtained formulation may be subjected to conventional after-treatments well known in the pharmaceutical technology, e.g. sterilization.

The amount of the active ingredient may be varied within a wide range, e.g. about from 0.01 to 95% w/w in these formulations.

A tablet formulation according to the invention may contain in addition to the active ingredient a filler such as microcrystalline cellulose and/or polyvinylpyrrolidone; a disintegrator, such as starch and/or carboxymethyl starch as well as an antiadhesion or lubricant such as magnesium stearate and/or talc.

A preferable capsule formulation according to the present invention may include an above mentioned inert filler, disintegrator and/or lubricant.

A preferable suspension formulation of the present invention can be prepared by evenly dispersing the finely divided active ingredient in a syrup containing a flavouring agent, e.g. raspberry or strawberry aroma; a colouring agent; a food dye; a viscosity increasing agent e.g. Carbopole; a wetting agent, e.g. Tween, as well as a preservative e.g. sodium benzoate.

According to a second aspect of the present invention,there is provided a method of treating or preventing ulceration in mammals inclusive men by using a compound of formula (I) wherein R and $R^1$ are as defined hereinbefore or a pharmaceutical composition containing such a compound in an amount sufficient to ensure the desired healing or preventing effect.

According to a third aspect of the present invention, there is provided the use of a compound of formula (I) wherein R and $R^1$ are as defined hereinbefore in the preparation of a pharmaceutical composition for the prophylaxis or treatment of ulceration in mammals, inclusive men.

The dosage regimen of the active ingredient may be varied within a wide range depending on various factors such as the nature of the active ingredient in question,the species, age and body weight of the subject to be treated, the severity and symptoms of the disease, etc. therefore the exact dose must be prescribed by the physician individually in each case. In general, the dosage may vary about from 10 to 200 mg active ingredient per day per adult in case of enteral administration.

For a better understanding of the invention, the following non-limiting Examples are given to illustrate the preparation of the pharmaceutical compositions of the invention.

Example I

Tablet formulation

The active ingredient was measured, sieved and homogenized with the fillers then admixed with the microcrystalline cellulose. The whole mixture was granulated with an aqueous solution of polyvinylpyrrolidone, then dried. The granulate first was mixed with the disintegrator then the whole mixture was homogenized with the antiadhesion agents and lubricants. The homogenous granules were compressed into tablets.

| Composition of tablets | | |
|---|---|---|
| Amount | Active ingredient | |
| | F | I |
| active ingredient | 10.0 mg | 20.0 mg |
| colloidal hydrophyl silica | 0.7 mg | 1.5 mg |
| magnesium stearate | 1.5 mg | 3.0 mg |
| polyvinylpyrrolidone | 3.0 mg | 6.0 mg |
| talc | 4.5 mg | 9.0 mg |
| sodium carboxymethylamylopect. | 6.0 mg | 12.0 mg |
| mycrocrystalline cellulose | 6.3 mg | 18.5 mg |
| corn starch | 40.0 mg | 80.0 mg |
| lactose | 78.0 mg | 150.0 mg |
| total weight | 150.0 mg | 300.0 mg |

Example II

Suspension formulation

Sugar and sterile water were cooked to give a syrup. Carbopole was swelled in sterile water then mixed with the syrup. The finely divided active ingredient was intimately admixed with the surface active agents then dispersed with the viscous syrup. The colouring and flavouring agent and preservative were dissolved in sterile water then admixed to the suspension. The suspension was homogenised than filled into ampulles or plastic containers while ensuring the homogenity of the mixture.

| Composition of suspension | | |
|---|---|---|
| Amount | Active ingredient | |
| | F | I |
| active ingredient | 1.00 g | 1.00 g |
| raspberry aroma | 1.00 g | 0.40 g |
| FD + C red No 40 | 0.03 g | 0.01 g |
| citric acid | 0.97 g | 0.33 g |
| sodium benzoate | 0.90 g | 0.30 g |
| polyacrylic acid | 1.20 g | 0.40 g |
| sorboxaethen olienicum | 0.10 g | 0.03 g |
| sucrose | 150.00 g | 50.00 g |
| distilled water | ad 300.00 g | ad 150.0 g |
| Suitable administration: | 50 mg/spoonful | 50 mg/teaspoonful |

Some preferable preparation methods for obtaining an active ingredient of formula (I) are illustrated by the following non-limiting Examples.

Example I

3-Phenylthio-2(Z)-propenoic acid

Propyolic acid (28 g, 0.4 mol) was dissolved in a 2.5N solution of sodium hydroxide (160 ml) under ice

water cooling, then thiophenol (44 g, 0.4 mol) was added dropwise to the solution. The reaction mixture was stirred for 2 hours, then diluted with water (200 ml) and the pH was adjusted to 1 with a 10% solution of hydrochloric acid. After stirring for 1 hour, the solid precipitate was filtered off, washed with water and dried. The crude product was recrystallised from carbon tetrachloride (300 ml) to give the title compound (40.3 g, 56%).
Melting point: 102 to 104 °C.
$R_f$: 0.60 in ethyl acetate/glacial acetic acid (40:1).

Example 2

3-Phenylthio-2(E)-propenoic acid

Propyolic acid (28 g, 0.4 mol) was dissolved in a 2.5N solution of sodium hydroxide (160 ml) under ice cooling, then thiophenol (44 g, 0.4 mol) was added dropwise to the solution. The reaction mixture was stirred for 2 hours at room temperature, then dichloromethane (400 ml) was added and the pH value of the solution was adjusted to 1 with a 10% solution of hydrochloric acid. The organic layer was separated, dried over anhydrous magnesium sulfate and the solvent was evaporated. The residue was dissolved in xylene (200 ml) and boiled for 10 hours, then crystallised under ice water cooling. After cooling for 3 hours the solid was filtered off, washed with xylene at 0 °C and dried to give the title compound (52.5 g, 73%).
Melting point: 127 to 129 °C
$R_f$:0.57 in ethyl acetate/glacial acetic acid (40 : 1).

The solvent was evaporated from the carbon tetrachloride mother liquor and the residue was boiled in xylene (50 ml) for 10 hours to give a further crop of the title compound (18 g, 25%) after cooling. Physical characteristics of this product were identical with those of the product obtained by the first crystallisation.

Example 3

3-Phenylsulfinyl-2(Z)-propenoic acid

3-Phenylthio-2(Z)-propenoic acid (5.4 g, 30 mmol) obtained in Example 1 was dissolved in glacial acetic acid (30 ml) and a 35% solution of hydrogen peroxide (3.5 ml) was added dropwise to the solution then the mixture was left to stand at room temperature for 3 days. After evaporation of the solvent, the solid residue was recrystallised twice from benzene to give the title compound (2.5 g, 42.5%).
Melting point: 121 to 124 °C.

Example 4

3-Phenylsulfinyl-2(E)-propenoic acid

A 35% solution of hydrogen peroxide (4 ml) was added to a solution of 3-phenylthio-2(Z)-propenoic acid (5.4 g, mmol) in glacial acetic acid (30 ml), then the mixture was stirred at 90 to 100 °C for 1.5 hours. After evaporation of the solvent, the solid residue was triturated with carbon tetrachloride, filtered off and washed with carbon tetrachloride to give the title compound (5.4 g, 91%).
Melting point: 134 to 136 °C.

Example 5

3-Phenylsulfonyl-2(Z)-propenoic acid

A 35% solution of hydrogen peroxide (4 ml) was added to a solution of 3-phenylthio-2(Z)-propenoic acid

(3.6 g, 30 mmol) in glacial acetic acid (30 ml), and the mixture was stirred at 90 to 100 °C for 1.5 hours. After evaporation of the solvent, the residue was suspended in carbon tetrachloride and filtered off to give the title compound (3.6 g, 85%).
Melting point: 163 to 166 °C.

Example 6

3-Phenylsulfonyl-2(E)-propenoic acid

A 35% solution of hydrogen peroxide (21 ml) was added to a solution of 3-phenylthio-2(E)-propenoic acid (18.0 g, 0.1 mol) in glacial acetic acid (125 ml) and the mixture was stirred at 100 °C for 2 hours. After evaporation of the solvent, the solid residue was recrystallised from toluene (150 ml) to give the title compound (18.7 g, 91%).
Melting point: 133 to 135 °C.

Example 7

Magnesium bis[3-phenylthio-2(E)-propenoate]

Triethylamine (7 ml, 0.05 mol) was added to a suspension of 3-phenylthio-2(E)-propenoic acid (9 g, 0.05 mol) obtained in Example 2 in water (50 ml). A solution of $MgCl_2.6H_2O$ (5.1 g, 0.025 mol) in water (20 ml) was added to the solution. After stirring for 1 hour, 3/4 parts of the water was evaporated then the salt was precipitated with acetonitrile (200 ml). The precipitate was filtered off, washed with acetonitrile and dried to give the title compound (7.5 g, 78.5%).
Melting point: 231 to 238 °C (with decomposition)
Elementary analysis:
%Mg calculated 6.35,
found 6.28.

Example 8

Magnesium bis[3-phenylsulfonyl-2(E)-propenoate] tetrahydrate

Triethylamine (2.1 ml, 15 mmol) was added to a suspension of 3-phenylsulfonyl-2(E)-propenoic acid in water (15 ml) to give a clear solution. A solution of $MgCl_2.6H_2O$ (1.52 g, 7.5 mmol) in water (5 ml) was added to the solution and the mixture was stirred for 30 minutes then 3/4 parts of the water was evaporated. Acetonitrile (50 ml) was added to the residue to give a white precipitate, which was filtered off and washed with acetonitrile to obtain the title compound (3.4 g, 87.4%).
Melting point: 190 to 192 °C (with decomposition)
Elementary analysis:
% Mg calculated 4.68
found 4.68.

Example 9

Methyl 3-phenylsulfonyl-2(E)-propenoate

A solution of 3-phenylsulfonyl-2(E)-propenoic acid (4.24 g, 20 mmol) in methanol (50 ml) was boiled in the presence of a 6N solution of hydrochloric acid in methanol (2.5 ml) for 2.5 hours. After evaporation of the methanol, the residue was dissolved in dichloromethane (25 ml) and extracted with a 5% solution of

sodium bicarbonate (3 x 5 ml). The organic layer was dried over anhydrous magnesium sulfate and the solvent was evaporated. The solid residue was suspended in diisopropyl ether, filtered off and washed with diisopropyl ether to give the title compound (3.7 g, 81%).
Melting point: 99.5 to 100.5 °C.

Example 10

3-(4-Chlorophenylthio)-2(E)-propenoic acid

Propyolic acid (14 g, 0.2 mol) was dissolved in a 10% solution of sodium hydroxide (80 ml) at 0 °C, then 4-chlorothiophenol (28.9 g, 0.2 mol) was added dropwise to the solution. The reaction mixture was stirred at room temperature for 10 hours, then diluted with dichloroethane (200 ml) and adjusted to pH 1 with a 10% solution of hydrochloric acid. The organic layer was dried over anhydrous magnesium sulfate, then evaporated and the residue was dissolved in xylene (150 ml). After boiling for 6 hours, the mixture was cooled to room temperature and the precipitate was filtered off, washed with petroleum ether (b.p. 70 °C) and dried to give the title compound (28 g, 65%).
Melting point: 118 to 120 °C.

Example 11

3-(4-Chlorophenylsulfonyl)-2(E)-propenoic acid

A 33% solution of hydrogen peroxide (25.4 g, 0.2 mol) was added to a solution of 3-(4-chlorophenylthio)-2(E)-propenoic acid (21.5 g, 0.1 mol) in glacial acetic acid (150 ml). The reaction solution was stirred at 90 to 100 °C for 2 hours, then the solvent was evaporated. The solid residue was recrystallised from glacial acetic acid to give the title compound (21 g, 85%).
Melting point: 189 to 190 °C.

**Claims**

1. A pharmaceutical composition **comprising** as active ingredient at least one compound of formula

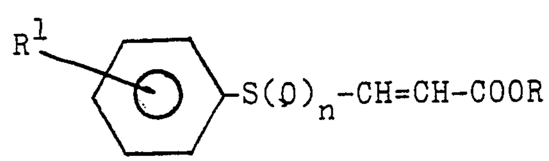

(I)

wherein
n is an integer of from 0 to 2,
R represents a hydrogen, an alkaline or an alkaline earth metal atom, or a $C_{1-4}$alkyl group, and $R^1$ represents a hydrogen or a halogen atom or a $C_{1-4}$-alkyl, a $C_{1-4}$alkoxy or a nitro group
and at least one pharmaceutical carrier and/or other excipient.
2. A pharmaceutical composition according to claim 1, **comprising** additionally at least one known active ingredient exerting no synergistic effect in the given combination.
3. A process for the preparation of a pharmaceutical composition, which **comprises** admixing a compound of formula

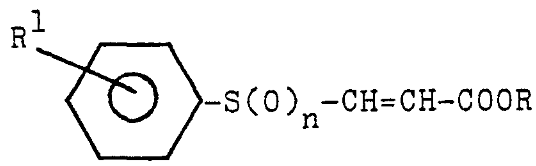

(I)

wherein
n is an integer of from 0 to 2,
R represents a hydrogen, an alkaline or an alkaline earth metal atom, or a $C_{1-4}$alkyl group, and
$R^1$ represents a hydrogen or a halogen atom or a $C_{1-4}$-alkyl, a $C_{1-4}$alkoxy or a nitro group,
with at least one pharmaceutical carrier and/or excipient and optionally with one or more known active ingredients exerting no synergistic effect in the given combination and converting the mixture into a pharmaceutical formulation.

4. A method for treating or preventing ulceration in the digestive tract of mammals inclusive human beings, which **comprises** using a compound of formula

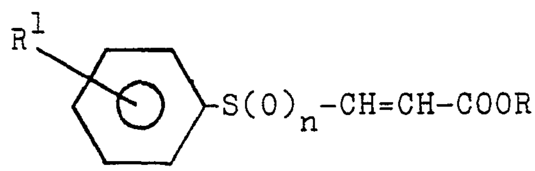

(I)

wherein
n is an integer of from 0 to 2,
R represents a hydrogen, an alkaline or an alkaline earth metal atom, or a $C_{1-4}$alkyl group, and
$R^1$ represents a hydrogen or a halogen atom or a $C_{1-4}$-alkyl, a $C_{1-4}$alkoxy or a nitro group,
or a pharmaceutical composition containing such a compound in an amount sufficient to ensure the desired healing or preventing effect.

5. The use of a compound of formula

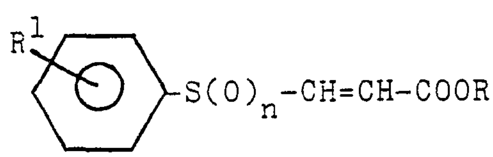

(I)

wherein
n is an integer of from 0 to 2,
R represents a hydrogen, an alkaline or an alkaline earth metal atom, or a $C_{1-4}$alkyl group, and
$R^1$ represents a hydrogen or a halogen atom or a $C_{1-4}$-alkyl, a $C_{1-4}$alkoxy or a nitro group,
in the preparation of a pharmaceutical composition for the prophylaxis and treatment of ulceration in the digestive tract of mammals, inclusive human beings.